## Europäisches Patentamt

## European Patent Office

## Office européen des brevets

(19)

(11) Veröffentlichungsnummer: **0 040 375 B1**

(12) **EUROPÄISCHE PATENTSCHRIFT**

(45) Veröffentlichungstag der Patentschrift:
**31.10.84**

(21) Anmeldenummer: **81103528.6**

(22) Anmeldetag: **08.05.81**

(51) Int. Cl.³: **G 10 K 11/34**

(54) Ultraschallwandleranordnung.

(30) Priorität: **21.05.80 DE 3019409**

(43) Veröffentlichungstag der Anmeldung:
**25.11.81 Patentblatt 81/47**

(45) Bekanntmachung des Hinweises auf die Patenterteilung:
**31.10.84 Patentblatt 84/44**

(73) Patentinhaber: **Siemens Aktiengesellschaft, Berlin und München Wittelsbacherplatz 2, D-8000 München 2 (DE)**

(72) Erfinder: **Diepers, Heinrich, Dr., Velt-Stoss-Strasse 44, D-8552 Höchstadt/Aisch (DE)**

(84) Benannte Vertragsstaaten:
**AT CH DE FR GB LI NL**

(56) Entgegenhaltungen:
**EP - A - 0 031 510**
**DE - B - 2 829 570**
**FR - A - 2 292 978**
**FR - A - 2 349 835**
**GB - A - 2 005 835**

**MEDICAL PHYSICS, Band 3, Nr. 5, 1976, Sept./Okt., Am. Inst. of Physics New York, US MAGINNESS et al.: "State-of-the-art in two-dimensional ultrasonic transducer array technology", Seite 312-318**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung bezieht sich auf ein Ultraschallwandler-Array mit einer Matrix von Ultraschallschwingern, die jeweils aus einer Submatrix von gemeinsam gesteuerten säulenförmigen Wandlerelementen bestehen.

Mit sogenannten Phased Arrays kann bekanntlich durch Änderung der Amplituden- und der Phasenansteuerung das abgestrahlte und empfangene Schallfeld so beeinflußt werden, daß die Wirkung eines Winkelprüfkopfes und eines fokussierenden Kopfes in einem einzigen Array-prüfkopf vereinigt werden kann. Die lineare Laufzeitverzögerung der Ultraschallimpulse ergibt ein Schwenken und durch eine symmetrische, beispielsweise quadratische Verzögerung der Ultraschallimpulse erhält man ein Fokussieren des Schallfeldes. Durch Überlagerung einer linearen mit einer quadratischen Verzögerung wird das Schallfeld in der Längsrichtung der Wandleranordnung sowohl fokussiert als auch geschwenkt. Mit einer als akustische Linse wirkenden Vorlaufstrecke kann das Schallfeld zusätzlich noch in der Ebene quer zur Längsrichtung der Wandleranordnung fokussiert werden. Die Fokussierung ist jedoch auf einen Wert begrenzt, der durch die Krümmung der Linse gegeben ist (US-A- 3 936 791).

Der Erfindung liegt nun die Aufgabe zugrunde, ein Ultraschallwandler-Array anzugeben, das wahlweise als Normalprüfkopf, Winkel- oder auch Fokusprüfkopf sowie als fokussierender Winkelprüfkopf eingesetzt werden kann und das für eine Verlagerung des Fokuspunktes in der Tiefe des zu untersuchenden Körpers geeignet ist.

Es ist bereits eine Ultraschallwandleranordnung vorgeschlagen worden, die aus einer Matrix von Ultraschallschwingern besteht, die jeweils eine Matrix von gemeinsam gesteuerten säulenförmigen Wandlerelementen enthalten. Auf einer Flachseite der Matrix sind die Wandlerelemente aller Ultraschallschwinger an einem gemeinsamen Steuerleiter angeschlossen. Dagegen sind auf der anderen Flachseite der Matrix die Schwinger getrennt steuerbar (EP-A-10 025 092).

In einer derartigen Matrix von Ultraschallschwingern, die an einer ihrer Stirnseiten mit einem gemeinsamen Steueranschluß und auf der anderen Stirnseite jeweils mit einem getrennten Steuerleiter verbunden sind, können durch entsprechende Steuerung konzentrische Gruppen von Schwingern gebildet werden, die dann zur Abtastung größerer Räume in Längsrichtung der Matrix fortgeschaltet werden (US-A-4 219 846).

Ferner ist eine seitliche Fortschaltung konzentrischer Schwingergruppen dadurch möglich, daß alle Schwinger an einer ihrer Stirnseiten getrennt steuerbar sind und daß an der anderen Stirnseite jeweils durch einen gemeinsamen Steueranschluß Reihen von Schwingern gebildet werden. Jeweils zwei symmetrisch zu einer mittleren Reihe liegende Reihen werden gemeinsam gesteuert (US-A-4 307 613).

Weiterhin sind Ring-Arrays bekannt, bei denen ringförmige Ultraschallschwinger unabhängig voneinander gesteuert werden und der Öffnungswinkel (Apertur) durch Zu- oder Abschalten von äußeren Ringen verändert und damit beim Senden der sogenannte natürliche Fokuspunkt verschoben wird. Man erhält eine sogenannte atmende Apertur. Beim Empfang wird durch kontinuierliche, elektronische Phasensteuerung ein dynamischer, in der Tiefenlage kontinuierlich verschiebbarer Fokuspunkt erzeugt. Diese dynamische Fokussierung erfordert jedoch einen verhältnismäßig großen elektronischen Aufwand (»Ultrasonic Imaging«, Vol. 1, No. 1, 1979, S. 56—75).

Die Erfindung beruht nun auf der Erkenntnis, daß mit einer besonderen Kontaktierung und Ansteuerung der Ultraschallschwinger innerhalb der Matrix in Verbindung mit der bekannten phasenverzögerten Ansteuerung die Funktion der verschiedenen Prüfköpfe in einem einzigen elektronischen Prüfkopf vereinigt werden kann. Die Erfindung besteht somit in den kennzeichnenden Merkmalen des Anspruchs 1. Die Vereinigung der verschiedenen Funktionen erhält man durch die besondere Kontaktierung der Ultraschallschwinger auf den beiden Flachseiten der Matrix in Verbindung mit der Möglichkeit, die Elektroden auf jeweils einer der Flachseiten in bestimmter Weise mit Erregerimpulsen zu beaufschlagen und auf der anderen Seite auf ein gemeinsames Potential, vorzugsweise Nullpotential, zu legen.

Werden die auf einer Flachseite der Matrix gruppenweise konzentrisch angeschlossenen Ultraschallschwinger auf Nullpotential gelegt und die Ultraschallschwinger auf der anderen Flachseite der Matrix zeilenweise gemeinsam unverzögert erregt, so erfolgt die Abstrahlung in das zu prüfende Werkstück senkrecht zu den Flachseiten der Matrix. Wird den Zeilen der Ultraschallschwinger jeweils ein Sender einer Senderkette und jeweils ein Verzögerungsglied einer linearen elektronischen Verzögerungskette vorgeschaltet und wird eine derartige lineare Verzögerungskette auch dem Empfänger zugeordnet, so kann nacheinander mit verschiedenen Winkeln in das Werkstück eingeschaltet werden. Die Größe des Winkels ist durch die Verzögerung bestimmt. Mit einer beispielsweise quadratischen Verzögerung erfolgt Sendung und Empfang senkrecht zur Matrix mit einem Fokus, der durch die Verzögerung eingestellt werden kann. Der Fokuspunkt kann somit in verschiedene Tiefen des zu untersuchenden Werkstücks gelegt werden. Mit der Überlagerung einer linearen und einer quadratischen Verzögerung erfolgt die Einschallung mit einstellbarer Richtung und einstellbarem Fokus, wobei der Abstrahlwinkel durch die Einstellung der linearen Verzögerung und die Fokustiefe durch die Einstellung der quadratischen Verzögerung gewählt werden kann.

Werden die Zeilen der Schwingerelektroden auf der einen Flachseite der Matrix des Wandlerarrays auf Nullpotential gelegt und die konzentrischen Elektrodenverbindungen auf der anderen Flachseite der Matrix mit einer quadratischen Verzögerung angesteuert, so erhält man bei senkrechter Abstrahlrichtung sowohl eine Fokussierung in der Ebene senkrecht zu den Elektrodenreihen der Ultraschallschwinger als auch in der Ebene in deren Längsrichtung. Man erhält somit durch diese räumliche Fokussierung einen Fokusschlauch.

Die Abmessungen der säulenförmigen Wandlerelemente werden vorzugsweise so gewählt, daß ihre Länge etwa gleich der halben Wellenlänge und damit etwa doppelt so groß wie ihre Dicke ist.

Zur weiteren Erläuterung der Erfindung wird auf die Zeichnung Bezug genommen, in deren

Fig. 1 ein Ultraschallwandlerarray nach der Erfindung mit seiner elektronischen Steuerung schematisch veranschaulicht ist. In

Fig. 2 ist ein Teil der Fig. 1 als Schnitt dargestellt.

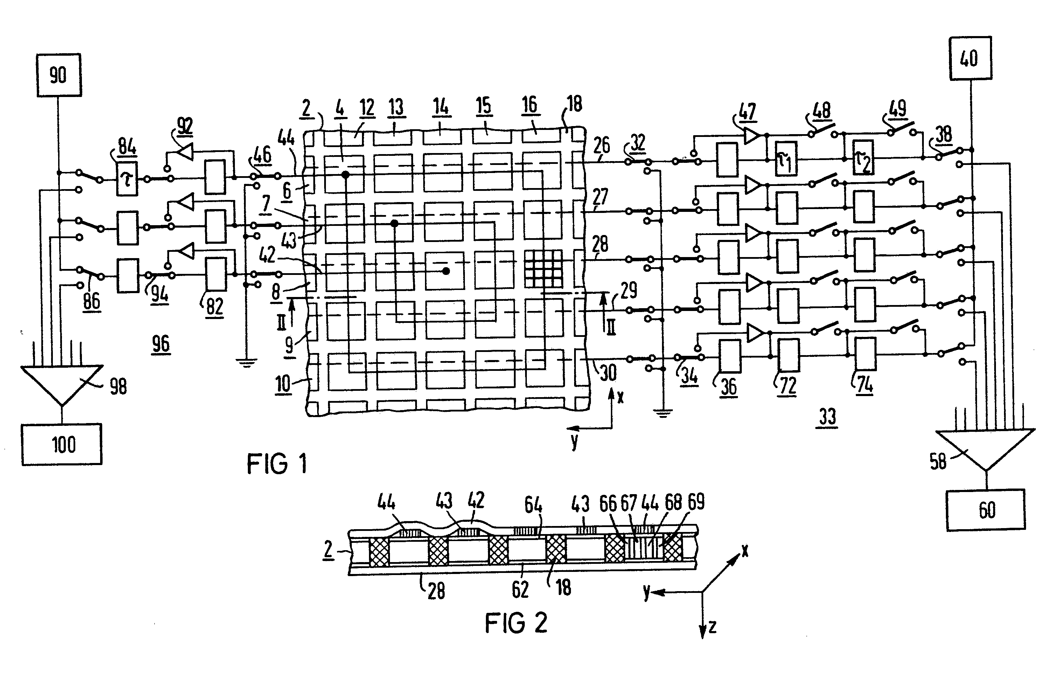

Nach Fig. 1 ist eine Matrix 2 von Ultraschallschwingern 4 in Zeilen 6 bis 10 hintereinander und in Spalten 12 bis 16 nebeneinander angeordnet. Die Zwischenräume 18 zwischen den Ultraschallschwingern 4 sind in der Figur zur Verdeutlichung vergrößert dargestellt. Die säulenartigen Ultraschallschwinger 4 sind innerhalb der Matrix derart angeordnet, daß ihre untere Stirnfläche in der unteren Flachseite der Matrix und ihre obere Stirnfläche in der oberen Flachseite der Matrix 2 liegt. An der unteren Flachseite der Matrix 2 sind die Ultraschallschwinger 4 der Zeilen 6 bis 10 jeweils über eine gemeinsame Steuerleitung an einem Sender und einem Empfänger angeschlossen. Die Steuerleiter sind in der Figur mit 26 bis 30 bezeichnet. Sie können über eine Umschalterkette 32, die vorzugsweise aus elektronischen Schaltern bestehen kann, auf Nullpotential gelegt werden.

Die Steuerleiter 26 bis 30 sind über eine Elektronik 33 mit dem Taktgeber 40 eines Senders und mit einem Summierverstärker 58 eines Empfängers 60 verbunden, der beispielsweise ein elektronischer Bildschirm sein kann. Die Elektronik 33 enthält für die Zeilen 6 bis 10 der Ultraschallschwinger 4 jeweils einen Schalter einer Schalterkette einer elektronischen Weiche 34 sowie einen Sender einer Senderkette 36 und einen Umschalter einer elektronischen Weiche 38. Die elektronische Weiche 34 und gegebenenfalls auch die Weiche 38 können vorzugsweise als integrierte Schaltkreise ausgebildet sein, deren Sperrdämpfung vorzugsweise wenigstens etwa 40 dB betragen kann.

An der oberen Flachseite der Matrix 2 ist ein nicht näher bezeichneter zentraler Ultraschallschwinger an einen Steuerleiter 42 angeschlossen. Jeweils Gruppen der Ultraschallschwinger 4, die den zentralen Ultraschallschwinger konzentrisch umgeben, sind an einem gemeinsamen Steuerleiter angeschlossen, die in der Figur mit 43 und 44 bezeichnet sind. Werden die Steuerleitungen 42 bis 44 der Elektroden an der oberen Flachseite der Matrix 2 über jeweils einen Umschalter einer gemeinsam mit 46 bezeichneten Umschalterkette auf Nullpotential gelegt, so können die Ultraschallschwinger 4 der Matrix 2 mit Hilfe des Taktgebers 40 und der Senderkette 36 über die Steuerleitungen 26 bis 30 zeilenweise angesteuert werden. Man erhält dann eine Abstrahlung senkrecht zu den Flachseiten der Matrix 2 in einem in der Figur nicht dargestellten zu prüfenden Körper. Nach Abgabe der Sendeimpulse wird die elektronische Weiche 34 umgeschaltet und die Echosignale werden im allgemeinen über den Vorverstärker einer Vorverstärkerkette 47 sowie die Reihenschaltung von zwei elektronischen Schaltern, die jeweils zu einer Kette von Überbrückungsschaltern 48 bzw. 49 gehören, und die umgeschaltete elektronische Weiche 38 dem Empfänger 60 zugeführt.

Die Ultraschallschwinger 2 können vorzugsweise durch sogenannte Feinunterteilung sowohl in der x- als auch in der y-Richtung jeweils in eine Matrix von säulenförmigen Wandlerelementen aufgeteilt sein, wie es in Fig. 1 zur Vereinfachung nur bei einem Ultraschallschwinger der Zeile 8 angedeutet ist. Diese Wandlerelemente sind dann an ihren Enden jeweils gemeinsam kontaktiert und elektrisch parallelgeschaltet.

Nach Fig. 2 sind die Ultraschallschwinger in Richtung der Zeilen 6 bis 10 derartig nebeneinander angeordnet, daß ihre Stirnflächen, die jeweils mit einer Metallauflage 62 bzw. 64 versehen sind, an einer Flachseite der Matrix 2 liegen. Von den durch Feinteilung hergestellten Wandlerelementen ist die vordere Reihe einer der Ultraschallschwinger dargestellt und diese Elemente sind mit 66 bis 69 bezeichnet. Die Zwischenräume 18 zwischen den Ultraschallschwingern und gegebenenfalls auch zwischen den Wandlerelementen 66 bis 69 sind mit einem Material gefüllt, dessen akustischer Widerstand wesentlich vom Widerstand des Schwingermaterials abweicht. An der unteren Flachseite sind die Wandlerelemente der Zeile 8 durch die Steuerleitung 28 verbunden. An der oberen Flachseite der Matrix 2 sind die Steuerleiter 42 bis 44 sichtbar, die an der Oberfläche entlanggeführt sind. Die elektrische Isolation der Steuerleiter 42 bis 44 gegeneinander ist in der Figur zur Vereinfachung nicht dargestellt.

Soll die Abstrahlung von der Matrix 2 in der xz-Ebene mit verschiedenen Winkeln gegenüber der Flächennormalen erfolgen, so erhalten die Steuerleitungen 26 bis 30 jeweils eine elektronische Verzögerungsstufe einer linearen Verzögerungskette 72, die bei geschlossenem Schalter 49 und geöffnetem Schalter 48 die Taktimpulse des Taktgebers 40 den Sendern der Senderkette 36 jeweils mit einer linearen Verzögerung zuführen und die Echoimpulse über die Schalter der elektronischen Weiche 34 sowie die Vorverstärker der Vorverstärkerkette 46 mit der gleichen Verzögerung dem Summierverstärker 58 des Empfängers 60 zuführen. Durch Änderung der linearen Verzögerung der Verzögerungskette 72 können verschiedene Abstrahlwinkel eingestellt werden und das Wandlerarray wirkt somit wie ein Winkelprüfkopf. Mit einer weiteren Verzögerungskette 74, deren Verzögerungsglieder auf eine quadratische Verzögerung eingestellt sind und denen die Überbrückungsschalter 49 zugeordnet sind, können die Ultraschallschwinger 4 der Matrix 2 mit quadratischer Verzögerung angesteuert und ihre Echoimpulse mit der gleichen Verzögerung dem Empfänger zugeführt werden. Zu diesem Zweck werden die Schalter der Überbrückungsschalterkette 48 geschlossen und die Schalter 49 geöffnet.

Werden die Überbrückungsschalter 48 und 49 geöffnet, so sind sowohl die lineare Verzögerungskette 72 als auch die quadratische Verzögerungskette 74 wirksam. Mit der Überlagerung einer linearen und quadratischen Verzögerung in der Ansteuerung der Zeilen 6 bis 10 von Ultraschallschwingern 4 der Matrix 2 ist sowohl ein Schwenken als auch eine Fokussierung des abgestrahlten Schallfeldes möglich.

In einer besonders vorteilhaften Ausführungsform können die konzentrischen Elektrodengruppen der oberen Flachseite der Matrix 2 über die Steuerleiter 42 bis 44 jeweils an einem Sender und einem Empfänger angeschlossen werden. Zu diesem Zweck enthalten die Steuerleitungen jeweils einen Sender einer Senderkette 82 und jeweils eine elektronische Verzögerungskette 84 mit quadratischer Verzögerung, welche über jeweils einen Umschalter einer elektronischen Weiche 86 sowohl an den Taktgeber 90 eines Senders als auch an den Summierverstärker 98 eines Empfängers 100 angeschlossen ist. Die Sendeimpulse werden vom Taktgeber 90 über die Senderkette 82 den Ultraschallschwingern 4 der jeweiligen Schwingergruppe mit einer Verzögerung zugeführt, die an den Verzögerungsgliedern der Verzögerungskette 84 eingestellt wird. An der unteren Flachseite der Matrix 2 sind die Zeilen 6 bis 10 der Ultraschallschwinger 4 über den Umschalter 32 auf Nullpotential gelegt. Die Echosignale werden über jeweils einen Vorverstärker einer Vorverstärkerkette 92 sowie eine weitere elektronische Weiche 94 und die gleichen Verzögerungsglieder der Verzögerungskette 84 sowie die entsprechend umgeschaltete elektronische Weiche 86 und den Summierverstärker 98 dem Bildschirm des Empfängers 100 zugeführt. Durch Änderung der Zeitverzögerung der Verzögerungskette 84 kann die Größe der Fokussierung und damit die Tiefe des Fokussierungspunktes innerhalb des zu prüfenden Objekts eingestellt werden.

Im Ausführungsbeispiel verbinden die Steuerleitungen 43 und 44 jeweils konzentrisch zueinander angeordnete quadratische Elektrodengruppen. Unter Umständen kann es zweckmäßig sein, durch entsprechende Kontaktierung ringförmige oder auch sternförmige Elektrodengruppen zu bilden. Mit solchen besonderen Formen der Anregungsgeometrie und damit der Apertur des Wandlerarrays können beispielsweise Nebenkeulen in der Abstrahlcharakteristik des Wandlerarrays unterdrückt werden.

Durch die besondere Art der Ansteuerung der Zeilen 6 bis 10 der Matrix 2 durch die Elektronik 33 erhält man einen strichförmigen Fokus der Schallkeule, wenn die Elektroden der oberen Flachseite der Matrix 2 auf Nullpotential liegen. Werden die Elektroden der unteren Flachseite der Matrix 2 auf Nullpotential gelegt und die Elektroden der oberen Flachseite durch die Elektronik 96 angesteuert, so erhält man einen punktförmigen Fokus der Schallkeule. Mit dieser Ausführungsform des Wandlerarrays kann somit der zu prüfende Körper unter verschiedenen Winkeln durchstrahlt werden und ein aufgefundener Fehler kann durch die besondere Anordnung der Ultraschallschwingergruppen in Verbindung mit der besonderen Ansteuerung durch entsprechende Einstellung der nahezu punktförmigen bzw. strichförmigen Fokussierung näher untersucht werden.

**Patentansprüche**

1. Ultraschallwandler-Array, mit einer Matrix (2) von Ultraschallschwingern (4), die jeweils aus einer Submatrix von gemeinsam gesteuerten säulenförmigen Wandlerelementen (66 bis 69) bestehen, dadurch gekennzeichnet, daß

a) auf einer Flachseite der Matrix (2) die Elektroden von in Reihenform (6 bis 10) angeordneten Ultraschallschwingern (4) jeweils an eine gemeinsame Steuerleitung (26 bis 30) angeschlossen sind, daß

b) auf der gegenüberliegenden Flachseite der Matrix (2) die Elektroden jeweils konzentrisch zueinander angeordneter Gruppen von Ultraschallschwingern (4) an eine gemeinsame Steuerleitung (42 bis 44) angeschlossen sind, und daß

c) den Steuerleitungen jeder Seite jeweils wenigstens eine elektronische Verzögerungskette (72, 74; 84) zugeordnet ist.

2. Ultraschallwandler-Array nach Anspruch 1, dadurch gekennzeichnet, daß Wandlerelemente (66 bis 69) vorgesehen sind, deren Länge wenigstens annähernd gleich der halben Wellenlänge ($\lambda/2$) des Ultraschalls ist und deren Breite höchstens die Hälfte ihrer Länge beträgt.

3. Ultraschallwandler-Array nach Anspruch 1 oder 2, dadurch gekennzeichnet, daß alle Steuerelektroden der Ultraschallschwinger (4) jeweils einer der Flachseiten der Matrix (2) an ein gemeinsames Potential anschließbar sind.

4. Ultraschallwandler-Array nach Anspruch 3, dadurch gekennzeichnet, daß den Steuerleitungen der beiden Flachseiten der Matrix (2) jeweils eine Sender- und Empfängeranordnung mit einer elektronischen Umschaltung (34, 38 bzw. 86, 94) zugeordnet ist.

5. Ultraschallwandler-Array nach einem der Ansprüche 1 bis 4, dadurch gekennzeichnet, daß sowohl die Steuerleitungen (26 bis 30) für die Elektroden auf der einen Flachseite der Matrix (2) über einen Umschalter (32) als auch die Steuerleitungen (42 bis 44) für die Elektroden auf der gegenüberliegenden Flachseite der Matrix (2) über einen Umschalter (46) an ein gemeinsames Potential anschließbar sind.

6. Ultraschallwandler-Array nach Anspruch 5, dadurch gekennzeichnet, daß den Steuerleitungen der Reihen (6 bis 10) von Ultraschallschwingern (4) je eine Reihenschaltung von Verzögerungsgliedern einer linearen elektronischen Verzögerungskette (72) und einer quadratischen elektronischen Verzögerungskette (74) zugeordnet ist.

7. Ultraschallwandler-Array nach Anspruch 6, dadurch gekennzeichnet, daß den Steuerleitungen der konzentrischen Gruppen von Ultraschallschwingern (4) jeweils eine quadratische elektronische Verzögerungskette (84) zugeordnet ist, die, elektronisch umschaltbar, sowohl an einen Ultraschallsender als auch an einen Ultraschallempfänger (100) anschließbar ist.

8. Ultraschallwandler-Array nach einem der Ansprüche 1 bis 7, dadurch gekennzeichnet, daß die konzentrischen Gruppen der Ultraschallschwinger (4) jeweils einen Ring bilden.

9. Ultraschallwandler-Array nach einem der Ansprüche 1 bis 7, dadurch gekennzeichnet, daß die konzentrischen Gruppen der Ultraschallschwinger (4) jeweils einen Stern bilden.

## Claims

1. An ultrasonic transducer array, with a matrix (2) of ultrasonic oscillators (4), each of which consists of a sub-matrix of commonly controlled, column-like transducer elements (66 to 69), characterised in that

a) on one flat side of the matrix (2) the electrodes of ultrasonic oscillators (4) forming a row (6 to 10) are each connected to a common control line (26 to 30), that

b) on the opposite flat side of the matrix (2) the electrodes of ultrasonic oscillators (4) arranged in respective concentric groups are each connected to a respective common control line (42 to 44), and that

c) the control lines of each side are each assigned at least one electronic delay chain (72, 74; 84).

2. An ultrasonic transducer array as claimed in claim 1, characterised in that transducer elements (66 to 69) are provided, the length of which is at least approximately equal to half the ultrasonic wavelength ($\lambda/2$) and whose width is at the maximum equal to half its length.

3. An ultrasonic transducer array as claimed in claim 1 or 2, characterised in that the ultrasonic oscillators (4) control electrodes on one of the flat sides of the matrix (2) can all be connected to a common potential in each case.

4. An ultrasonic transducer array as claimed in claim 3, characterised in that the control lines of the two flat sides of the matrix (2) are each assigned a transmitter and a receiver arrangement with an electronic switch-over device (34, 38 or 86, 94 as the case may be).

5. An ultrasonic transducer array as claimed in one of the claims 1 to 4, characterised in that both the control lines (26 to 30) for the electrodes on one flat side of the matrix (2) and also the control lines (42 to 44) for the electrodes on the opposite flat side of the matrix (2) can be connected to a common potential in the former case via a change-over switch (32) and in the latter case via a change-over switch (46).

6. An ultrasonic transducer array as claimed in claim 5, characterised in that the control lines of the rows (6 to 10) of ultrasonic oscillators (4) are assigned a series arrangement of delay elements of a linear electronic delay chain (72) and of a quadratic electronic delay chain (74).

7. An ultrasonic transducer array as claimed in claim 6, characterised in that the control lines of the concentric groups of ultrasonic oscillators (4) are each assigned a quadratic electronic delay chain (84) which can be switched over electronically to connect an ultrasonic transmitter or an ultrasonic receiver (100).

8. An ultrasonic transducer array as claimed in one of the claims 1 to 7, characterised in that the concentric groups of the ultrasonic oscillators (4) each form a ring.

9. An ultrasonic transducer array as claimed in one of the claims 1 to 7, characterised in that the concentric groups of the ultrasonic oscillators (4) each form a star.

## Revendications

1. Transducteur ultrasonique comportant une matrice (2) d'oscillateurs ultrasonores (4) dont chacun est constitué par une sous-matrice d'éléments transducteurs (66 à 69) en forme de colonnes et commandés en commun, caractérisé par le fait que

a) sur un côté plat de la matrice (2), les électrodes des oscillateurs ultrasonores (4) qui sont disposés en rangées (6 à 10) sont respectivement reliées à un conducteur commun de commande (26 à 30), que

b) sur le côté plat opposé de la matrice (2), les électrodes respectives des groupes d'oscillateurs ultrasonores (4) qui sont disposés concentriquement entre eux, sont reliées à un conducteur commun de commande (42 à 44), et que

c) aux conducteurs de commande de chaque côté est respectivement associée au moins une chaîne électronique à retard (72, 74; 84).

2. Transducteur ultrasonique selon la revendication 1, caractérisé par le fait que sont prévus des éléments transducteurs (66 à 69) dont les longueurs sont au moins approximativement égales à la demilongueur d'onde ($\lambda$/2) de l'ultrason et dont les largeurs sont au maximum égales à la moitié de leur longueur.

3. Transducteur ultrasonique selon la revendication 1 ou 2, caractérisé par le fait que toutes les électrodes de commande des oscillateurs ultrasonores (4) respectivement d'un des côtés plats de la matrice (2) sont susceptibles d'être portées à un potentiel commun.

4. Transducteur ultrasonique selon la revendication 3, caractérisé par le fait qu'à chacun des conducteurs de commande des deux côtés plats de la matrice (2) est respectivement associé un dispositif émetteur et récepteur, avec un commutateur électronique (34, 38 ou 86, 94).

5. Transducteur ultrasonique selon l'une des revendications 1 à 4, caractérisé par le fait qu'aussi bien les conducteurs de commande (26 à 30) pour les électrodes situées sur un côté plat de la matrice (2) que les conducteurs de commande (42 à 44) pour les électrodes situées sur le côté plat opposé de la matrice (2), sont susceptible d'être portés à un potentiel commun respectivement par l'intermédiaire d'un commutateur (32) et d'un commutateur (46).

6. Transducteur ultrasonique selon la revendication 5, caractérisé par le fait qu'aux conducteurs de commande des rangées (6 à 10) d'oscillateurs ultrasonores (4) sont respectivement associés un montage série constitué par des éléments à retard d'une chaîne à retard linéaire électronique (72) et d'une chaîne à retard électronique quadratique (74).

7. Transducteur ultrasonique selon la revendication 6, caractérisé par le fait qu'à chacun des conducteurs de commande des groupes concentriques d'oscillateurs ultrasonores (4) est associée une chaîne à retard électronique quadratique (84) qui est susceptible d'être commutée par voie électronique aussi bien sur un émetteur ultrasonore que sur un récepteur ultrasonore (100).

8. Transducteur ultrasonique selon l'une des revendications 1 à 7, caractérisé par le fait que les groupes concentriques d'oscillateurs ultrasonores (4) forment chacun un anneau.

9. Transducteur ultrasonique selon l'une des revendications 1 à 7, caractérisé par le fait que les groupes concentriques des oscillateurs ultrasonores (4) forment chacun une étoile.

FIG 1

FIG 2

0 040 375